Europäisches Patentamt

European Patent Office

Office européen des brevets

(19)

(11)  EP 0 491 361 B1

(12)  EUROPÄISCHE PATENTSCHRIFT

(45) Veröffentlichungstag und Bekanntmachung des Hinweises auf die Patenterteilung:
17.03.1999   Patentblatt 1999/11

(51) Int Cl.⁶: **C07K 5/08**, A61K 38/06

(21) Anmeldenummer: 91121624.0

(22) Anmeldetag: 17.12.1991

(54) **Kurze Peptide mit Insulinwirkung**

Short peptides having the effect of insulin

Peptides brefs avec l'effet d'insuline

(84) Benannte Vertragsstaaten:
AT BE CH DE DK ES FR GB GR IT LI LU NL SE

(30) Priorität: 19.12.1990  DE 4040574

(43) Veröffentlichungstag der Anmeldung:
24.06.1992   Patentblatt 1992/26

(73) Patentinhaber: HOECHST AKTIENGESELLSCHAFT
65926 Frankfurt am Main (DE)

(72) Erfinder:
• Müllner, Stefan, Dr.
W-6203 Hochheim am Main (DE)
• König, Wolfgang, Dr.
W-6238 Hofheim am Taunus (DE)

• Müller, Günter, Dr.
W-6231 Sulzbach (DE)

(56) Entgegenhaltungen:
DD-B- 147 942          DE-A- 2 740 406
DE-A- 2 801 175

• CHEMICAL ABSTRACTS, Band 91, Nr. 23, 3. Dezember 1979, Columbus, Ohio, USA BERNDT, HEINZ "Synthesis of the human proinsulin sequence 71-86. III. Synthesis via the fragments 71-78 and 79-86." Seite 680, Spalte 1, Zusammenfassung-Nr. 193 628w
• CHEMICAL ABSTRACTS, Band 93, Nr. 11, 15. September 1980, Columbus, Ohio, USA EISLER, K. et al. "Syntesis and biological activity of five D-Cys-analogs of human insulin." Seite 780, Spalte 2, Zusammenfassung-Nr. 114 962r

**Beschreibung**

Die Erfindung betrifft neue Peptide mit Insulinwirkung, die sich zur Behandlung des Diabetes mellitus eignen.

Insuline bestehen aus zwei Polypeptidketten, der A-Kette, die 21 Aminosäurereste enthält und der B-Kette mit 30 Aminosäureresten. A- und B-Kette sind über zwei Disulfidbrücken miteinander verbunden, wobei die Cysteinreste in Position A7 und B7 sowie A20 und B19 miteinander verknüpft sind. Eine dritte Disulfidbrücke besteht zwischen A6 und A11. Tierische und menschliche Insuline werden in den Pankreata in Form von Präproinsulinen gebildet. Menschlichem Präproinsulin besteht z.B. aus einem Präpeptid mit 24 Aminosäureresten, daran anschließend ein Proinsulin mit 86 Aminosäureresten mit der folgenden Konfiguration: Präpeptid-B-Arg-Arg-C-Lys-Arg-A, wobei C für eine Aminosäurekette mit 31 Resten steht. Während der Exkretion aus den Langerhansschen Inseln wird das Präpeptid abgespalten und es entsteht Proinsulin. Abschließend wird die C-Kette proteolytisch gespalten und es entsteht das wirksame menschliche Insulin.

Insulin übt eine Vielzahl von Wirkungen auf insulinsensitives Gewebe aus. Ein auffälliger Effekt ist die schnelle Reduktion des Glucosespiegels in Säugetieren, wenn Insulin angewendet wird. Dies wird durch eine schnelle Aufnahme der Glucose aus dem Blut durch Muskel- und Fettzellen bewirkt. Insulin aktiviert ferner die Glycogen-Synthetase und inhibiert die Lipolyse. Insulin fördert die Proteinsynthese aus Aminosäuren und verstärkt die Induktion von Glycokinase und Phosphofructokinase und inhibiert die Bildung von bestimmten Enzymen der Gluconeogenese, wie Pyruvatcarboxylase und Fructosediphosphatase.

Der Diabetes Typ II, nicht insulinabhängiger Diabetes, geht einher mit einer Insulin-Resistenz der peripheren Gewebe wie Muskel- oder Fettgewebe. Die dadurch reduzierte Glukoseverwertung wird verursacht durch fehlende Isulin-Stimulation des Glucosetransports und nachfolgender metabolischer Prozesse. Diese multiple Resistenz läßt auf einen Defekt auf Rezeptor- oder Post-Rezeptor-Ebene, d.h, vor der Erzeugung der "second messenger" schließen (Garvey, Diabetes/Metabolism Reviews, 5, (1989), 727 - 742).

In dem Dokument DE 27 40 406 werden teilgeschützte Human-Insulin-A-Ketten beschrieben und Verfahren zu ihrer Herstellung. In dem Dokument DE 28 01 175 werden Verfahren zur Herstellung von cysteinhaltiger Peptide beschrieben.

Überraschenderweise wurde nun gefunden, daß kurze Peptide Insulinwirkung haben können und sich zur Behandlung des Diabetes mellitus eignen.

Die Erfindung betrifft somit Peptide der Formel II,

$$X\text{-}Q\text{-}Cys\text{-}D \hspace{10em} (II)$$

wobei

Q    für Tyr, Tyr($R^1$), Phe($R^2$), Trp($R^2$) oder Nal,

D    für Asp, Asn, D-Asp, D-Asn, beta-Ala oder Azagly-$NH_2$ und

X    für

        a) Wasserstoff,
        b) $R^1$-CO,
        c) ($C_1$-$C_{18}$)-Alkoxy-CO,
        d) ($C_3$-$C_{18}$)-Cycloalkoxy-CO oder
        e) ($C_6$-$C_{14}$)Aryl-($C_1$-$C_8$)-Alkoxy-CO, steht

$R^1$    für

        1.1 ($C_1$-$C_{18}$)-Alkyl,
        1.2 ($C_3$-$C_{18}$)-Cycloalkyl,
        1.3 ($C_6$-$C_{14}$)-Aryl,
        1.4 ($C_6$-$C_{14}$)-Aryl, ein- oder mehrfach substituiert durch 1.4.1 ($C_1$-$C_8$)-Alkyl, oder
        1.5 ($C_1$-$C_8$)-Alkyl, ein oder mehrfach substituiert durch 1.5.1 ($C_6$-$C_{14}$)-Aryl, steht,

$R^2$    für

        2.1 ($C_1$-$C_{18}$)-Alkyl,
        2.2 ($C_3$-$C_{18}$)-Cycloalkyl,
        2.3 ($C_1$-$C_{18}$)-Alkoxy,

2.4 $(C_3-C_{14})$-Cycloalkoxy,

2.5 $(C_6-C_{14})$-Aryl,

2.6 $(C_6-C_{14})$-Aryl, ein- oder mehrfach substituiert durch 2.6.1 $(C_1-C_8)$-Alkyl, 2.6.2 $(C_1-C_8)$-Alkoxy,

2.7 $(C_6-C_{14})$-Aryloxy,

2.8 $(C_6-C_{14})$-Aryloxy, ein- oder mehrfach substituiert durch 2.8.1 $(C_1-C_8)$-Alkyl,
2.8.2 $(C_1-C_8)$-Alkoxy,

2.9 $(C_1-C_8)$-Alkyl, ein- oder mehrfach substituiert durch 2.9.1 $(C_6-C_{14})$-Aryl,

2.10 $(C_6-C_8)$-Alkoxy, ein- oder mehrfach substituiert durch 2.10.1 $(C_6-C_{14})$-Aryl,

2.11 Halogen wie Fluor, Chlor, Brom oder Jod,

2.12 Nitro oder

2.13 Trifluormethyl, steht, oder

Dimere der Peptide der Formel I mit Cystin als Dimerisierungs-Komponente, ihre gegebenenfalls stereoisomeren Formen oder physiologisch verträgliche Salze des Peptids der Formel I, und die Verbindung Asn-Tyr-Cys-Asn-OH.

Insbesondere bevorzugt sind die Peptide
Asn Tyr Cys Asn oder
Tyr Cyr Asn.

Mit dem Begriff Aminosäuren sind z.B. die stereoisomeren Formen, d.h. D- oder L-Formen folgender Verbindungen gemeint:

| Alanin | Glycin | Prolin |
|---|---|---|
| Cystein | Histidin | Glutamin |
| Asparaginsäure | Isoleucin | Arginin |
| Glutaminsäure | Lysin | Serin |
| Phenylalanin | Leucin | Threonin |
| | Methionin | Valin |
| | Asparagin | Tryptophan |
| | | Tyrosin |
| 2-Amimoadipinsäure | | 2-Aminoisobuttersäure |
| 3- Aminoadipinsäure | | 3-Aminoisobuttersäure |
| beta-Alanin | | 2-Aminopimelinsäure |
| 2-Aminobuttersäure | | 2,4-Diaminobuttersäure |
| 4-Aminobuttersäure | | Desmosin |
| Piperidinsäure | | 2,2-Diaminopimelinsäure |
| 6-Aminocapromsäure | | 2,3-Diaminopropionsäure |
| 2-Aminoheptansäure | | N-Ethylglycin |
| 2-(2-Thienyl)-glycin Penicillamin | | 3-(2-Thienyl)-alanin |
| N-Ethylasparagin | | Sarkosin |
| Hydroxylysin | | N-Methylisoleucin |
| allo-Hydroxylysin | | 6-N-Methyllysin |
| 3-Hydroxyprolin | | N-Methylvalin |
| 4-Hydroxyprolin | | Norvalin |
| Isodesmosin | | Norleucin oder |
| allo-Isoleucin | | Ornithin |
| N-Methylglycin | | |

Die Kurzschreibweise der Aminosäuren erfolgt nach der allgemein üblichen Schreibweise (vgl. Schröder, Lübke, The Peptides, Band I, New York 1965, Seiten XXII - XXIII; Houben-Weyl, Methoden der Org. Chemie, Band XV/1 und 2 Stuttgart 1974).

Die Aminosäure pGlu steht für Pyroglutamyl, Nal für 3-(2-Naphthyl)-alanin, Azagly-NH$_2$ für eine Verbindung der Formel NH$_2$-NH-CONH$_2$ und D-Asp für die D-Form von Asparaginsäure. Peptide sind ihrer chemischen Natur nach Säureamide und zerfallen bei der Hydrolyse in Aminosäuren.

Unter Cycloalkyl werden auch Alkyl substituierte Reste, wie z.B. 4-Methylcyclohexyl oder 2,3-Dimethylcyclopentyl verstanden.

C$_6$-C$_{14}$-Aryl ist beispielsweise Phenyl, Naphthyl, Biphenylyl oder Fluorenyl; bevorzugt sind Phenyl und Naphthyl. Entsprechendes gilt auch für davon abgeleitete Reste, wie z.B. Aryloxy, Aralkyl und Aralkoxy. Unter Aralkyl versteht man z.B. einen mit C$_1$-C$_8$-Alkyl verknüpften unsubstituierten oder substituierten C$_6$-C$_{14}$-Arylrest, wie z.B. Benzyl, 1- und 2-Naphthylmethyl, Halobenzyl und Alkoxybenzyl, wobei Aralkyl jedoch nicht auf die genannten Reste beschränkt wäre.

Unter dem Begriff Alkyl werden geradgettige oder verzweigte Kohlenwasserstoffketten verstanden. Entsprechendes gilt für davon abgeleitete Reste, wie z.B. Alkoxy, Aralkyl und Alkanoyl.

Unter physiologisch verträglichen Salzen der Verbindung der Formel II sind insbesondere pharmazeutisch verwendbare oder nicht-toxische Salze zu verstehen. Solche Salze werden z.B. von Verbindungen der Formel II, welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin und Tris-(2-hydroxy-ethyl)-amin. Verbindungen der Formel II, welche basische Gruppen, z.B. eine Aminogruppe oder eine Guanidinogruppe enthalten, bilden mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure Salze. Verbindungen, In denen basische und sauren Gruppen in gleicher Zahl vorliegen, bilden innere Salze und sind auf eine dritte Salzkomponente nicht angewiesen.

Die Erfindung betrifft weiterhin ein Verfahren zur Herstellung von Peptiden der Formel II, das dadurch gekennzeichnet ist, daß an

a) ein Segment mit C-terminaler freier Carboxylgruppe oder dessen aktiviertes Derivat mit einem entsprechenden Segment mit N-terminaler freier Aminogruppe umsetzt oder
b) das Peptid stufenweise aufbaut,
in der nach (a) oder (b) erhaltenen Verbindung gegebenenfalls eine oder mehrere zum Schutz anderer Funktionen temporär eingeführte Schutzgruppen abspaltet und die so erhaltenen Verbindungen der Formel II gegebenenfalls in ihr physiologisch verträgliches Salz überführt.

Die erfindungsgemäßen Peptide werden nach den allgemeinen Methoden der Peptidchemie stufenweise vom C-terminalen Ende er oder durch Kupplung von Segmenten hergestellt (Houben-Weyl, Methoden der Organischen Chemie, Band 15/1,2). Die Peptidkupplungen können z.B. nach der Methode der gemischten Anhydride, über Aktivester, Azide oder nach der Carbodiimid-Methode, insbesondere unter Zusatz reaktionsbeschleunigender und racemisierungsverhindernder Substanzen wie z.B. 1-Hydroxybenzotriazol, N-Hydroxysuccinimid, 3-Hydroxy-4-oxo-3,4-dihydro-1.2.3-benzotriazin, N-Hydroxy-5-norbornen-2,3-dicarboximid, ferner unter Verwendung aktiver Derivate des 1-Hydroxybenzotriazols oder Anhydriden von Phosphor-, Phosphon- und Phosphinsäuren bei einer Reaktionstemperatur zwischen -10°C und dem Siedepunkt des Lösungsmittels, vorzugsweise zwischen -5°C und 40°C durchgeführt werden.

Geeignete Lösungsmittel dafür sind Dimethylformamid, Dimethylacetamid, N-Methylpyrrolidon oder Dimethylsulfoxid.

Sofern die Löslichkeit der Komponenten es erlaubt, können auch Lösungsmittel wie Methylenchlorid, Chloroform oder Tetrahydrofuran eingesetzt werden. Die genannten Methoden sind z.B. in Meienhofer-Gross: "The Peptides" Academic Press, Vol. I, (1979) beschrieben.

Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. T.W. Greene, "Protective Groups in Organic Synthesis") zusätzlich geschützt, wobei in erster Linie Arg(Tos), Arg(Mts), Arg(Mtr), Arg(PMC), Asp(OBzl), Asp(OBut), Cys-(4-MeBzl), Cys(Acm), Cys(SBut), Glu(OBzl), Glu(OBut), His(Tos), His(Fmoc), His(Dnp), His(Trt), Lys(Cl-Z), Lys(Boc), Met(O), Ser(Bzl), Ser(But), Thr(Bzl), Thr(But), Trp(Mts), Trp(CHO), Tyr(Br-Z), Tyr(Bzl) oder Tyr(But) eingesetzt werden.

Als Aminoschutzgruppen werden bevorzugt der durch katalytische Hydrierung abspaltbare Benzyloxycarbonyl-(Z-)Rest, der durch schwache Säuren abspaltbare 2-(3,5-Dimethyloxyphenyl)propyl(2)oxycarbonyl (Ddz-) oder Trityl- (Trt)-Rest und der durch sekundäre Amine abspaltbare 9-Fluorenylmethyloxycarbonyl- (Fmoc)- Rest herangezogen. Die SH-Gruppe des Cysteins kann durch eine Reihe von Schutzgruppen blockiert sein. Bevorzugt wird hier der Trityl- (Trt-) Rest und der S-tert.-Butyl-(StBu-) Rest. Der Tritylrest kann durch Jod-Oxydation unter Bildung der Cystin-Verbindungen oder durch reduzierende saure Spaltung zu den Cystein-Verbindungen abgespalten werden (Liebigs Ann. Chem. 1979, 227-247).

Der S-tert.-Butyl-Rest wird dagegen am besten mit Tributylphosphin reduktiv gespalten (Aust. J. Chem. 19 (1966) 2355-2360). OH- und COOH-Funktionen in den Seitenketten werden am besten durch den sauer abspaltbaren tert.-Butyl- (tBu-) Rest geschützt (Siehe auch: Meienhofer-Gross: "The Peptides, Vol. 3).

Die Verbindungen der Formel II und deren physiologisch verträgliche Salze dienen in erster Linie als Wirkstoffe für pharmazeutische Zubereitung zur Behandlung des Diabetes mellitus oder nicht insulinabhängiger Diabetes.

Erfindungsgegenstand ist daher auch eine pharmazeutische Zubereitungen, die durch einen Gehalt an mindestens einer Verbindung der Formel II und/oder mindestens einem von dessen physiologisch verträglichen Salzen in gelöster, amorpher und/oder kristalliner - vorzugsweise in amorpher und/oder kristalliner Form gekennzeichnet ist, wobei die Verbindungen H-Leu-Glu-Asn-Tyr-Cys-Asn-OH.
und H-Tyr-Gln-Leu-Glu-Asn-Tyr-Cys-Asn-OH nicht ausgenommen sind.

Für diese pharmazeutische Zubereitung bevorzugt sind die Peptide
Asn Tyr Cys Asn, H-Tyr-Gln-Leu-Glu-Asn-Tyr-Cys-Asn-OH oder Tyr Cys Asn und/oder
deren physiologisch verträgliche Salze.

Die pharmazeutische Zubereitung ist vorzugsweise eine Lösung oder Suspension zu Injektionszwecken mit einem pH-Wert zwischen etwa 3,0 und 9,0, vorzugsweise zwischen etwa 5,0 und 8,5 welche ein geeignetes Isotoniemittel, ein geeignetes Konservierungsmittel und gegebenenfalls einen geeigneten Puffer, sowie gegebenenfalls auch ein Depotprinzip, alles natürlich in steriler wäßriger Lösung oder Suspension, enthält. Die Gesamtheit der Zubereitungsbestandteile außer dem Wirkstoff bildet den Zubereitungsträger.

Geeignete Isotoniemittel sind z.B. Glycerin, Glukose, Mannit, NaCl, Calcium- oder Magnesium-Verbindungen wie z,B. $CaCl_2$ oder $MgCl_2$.

Geeignete Konservierungsmittel sind z.B. Phenol, m-Cresol, Benzylalkohol und/oder p-Hydroxybenzoesäureester.

Als Puffersubstanzen, insbesondere zur Einstellung eines pH-Wertes zwischen etwa 5,0 und 8,5 können z. B. Natriumacetat, Natriumcitrat, Natriumphosphat etc. verwendet werden. Ansonsten sind zur Einstellung des pH-Wertes auch physiologisch unbedenkliche verdünnte Säuren (typischerweise HCl) bzw. Laugen (typischerweise NaOH) geeignet.

Zwecks Variation des Wirkungsprofils der erfindugnsgemäßen Zubereitung können auch modifizierte (vgl. EP-B 132 769 und EP-B 132 770) und/oder unmodifizierte Insuline, vorzugsweise Rinder-, Schweine- oder Humaninsulin, insbesondere Humaninsulin, zugemischt werden.

Die pharmazeutische Zubereitung wird dadurch hergestellt, daß man mindestens eine Verbindung der Formel II und/oder mindestens eines von dessen physiologisch verträglichen Salzen, gegebenenfalls zusammen mit modifizierten und/oder unmodifizierten Insulin(derivat)en, mit einem physiologisch unbedenklichen Träger sowie gegebenenfalls mit geeigneten Zusatz- und Hilfsstoffen in eine geeignete Darreichungsform bringt.

Die Erfindung wird nun durch die folgenden Beispiele näher erläutert.

Beispiel 1:

H-Tyr-Cys-Asn-OH

1a. Fmoc-Tyr(tBu)-Cys(Trt)-Asn-OtBu

Zu einer Lösung von 3,4 g Fmoc-Tyr(tBu)-OH, 3,95 g (7,4 mmol) H-Cys(Trt)-Asn-OtBu (Liebigs Ann. Chem. 1979, 242), und 1 g HOBT in 50 ml Dimethylformamid gibt man bei 0°C und unter Rühren 1,63 g DCC und läßt 1 Std. bei 0°C rühren und über Nacht bei Raumtemperatur stehen. Am nächsten Tag wird der Niederschlag abgesaugt und das Filtrat eingeengt. Der Rückstand wird mit Essigester verrieben. Ausb. 2,83 g. Aus der Mutterlauge lassen sich nochmals 3,63 g isolieren. Gesamtausbeute: 6,46 g (89 %).
$C_{58}H_{62}N_4O_8S$ (975.218)
Schmp. 112-114°C, $[\alpha]_D^{21}$ = -15,3° (c=1, in Methanol).

1b. H-Tyr(tBu)-Cys(Trt)-Asn-OtBu

6 g (6,15 mol) Fmoc-Tyr(tBu)-Cys(Trt)-Asn-OtBu werden in 100 ml Dimethylformamid gelöst. Dazu gibt man 6,8 g Diethylamin und läßt 10 Minuten bei Raumtemperatur stehen. Anschließend wird im Hochvakuum eingeengt und der Rückstand in Methylenchlorid über Kieselgel chromatographiert. Mit Methylenchlorid wird lipophiler Dreck eluiert. Mit Methylenchlorid/Methanol wie 9,5:0,5 wird die Substanz eluiert. Ausb. 4,4 g Öl (95 %).
$C_{43}H_{52}N_4O_6S$ (752.976)

1c. H-Tyr-Cys-Asn-OH

2,2 g (2,9 mmol) H-Tyr(tBu)-Cys(Trt)-Asn-OtBu werden in einer Mischung aus 25 ml Trifluoressigsäure und 25 ml Ethylmercaptan gelöst. Nach 4 Stunden schüttet man den Ansatz in 250 ml Wasser. Die wässrige Lösung wird 3 mal mit Ether extrahiert und gefriergetrocknet. Ausb. 1,05 g (91 %).
$C_{16}H_{22}N_4O_6S$ (398.44)
$[\alpha]_D^{23}$ = -1,1° (c=1 in Wasser)

Beispiel 2:

H-Asn-Tyr-Cys-Asn-OH

2a. Fmoc-Asn-Tyr(tBu)-Cys(Trt)-Asn-OtBu.

Zu einer Lösung von 2,2 g (2,9 mmol) H-Tyr(tBu)-Cys(Trt)-Asn-OtBu, 1,04 g Fmoc-Asn-OH, 0,39 g HOBt in 30 ml Dimethylformamid gibt man unter Rühren bei 0°C 0,64 g DCC. Man läßt 1 Std bei 0°C rühren und läßt über Nacht bei Raumtemperatur stehen. Anderntags wird der Niederschlag abgesaugt und das Filtrat eingeengt. Der Rückstand wird in Essigester gelöst und nacheinander mit Wasser, ges. NaHCO$_3$-Lösung, KHSO$_4$-Lösung und Wasser extrahiert, über Na$_2$SO$_4$ getrocknet und eigeengt. Der Rückstand wird mit Petroläther verrieben. Ausb. 1,98 g (63 %).
$C_{62}H_{68}N_6O_{10}S$ (1089.323), $[\alpha]_D^{22}$ = -18,8° (c=1, in Methanol)

2b. H-Asn-Tyr(tBu)-Cys(Trt)-Asn-OtBu

1,9 g (1,74 mmol) Fmoc-Asn-Tyr(tBu)-Cys(Trt)-Asn-OtBu werden in 50 ml Dimethylformamid gelöst. Dazu gibt man 1,8 ml Diethylamin und läßt 15 Minuten bei Raumtemperatur stehen. Danach wird im Hochvakuum eingeengt und der Rückstand mit Essigester verrieben und im Vakuum getrocknet. Ausb. 0,98 g (65 %).
$C_{47}H_{58}N_6O_8S$ (867.081), $[\alpha]_D^{21}$ = -7,2° (c=1, in Methanol)

2c. H-Asn-Tyr-Cys-Asn-OH

0,9 g (1 mmol) der oben gewonnenen Verbindung werden in einer Mischung aus 10 ml Ethylmercaptan und 10 ml Trifluoressigsäure gelöst. Nach 4 Stunden wird die Mischung in 100 ml Wasser gegossen. Die wässrige Lösung wird 3 mal mit Ether extrahiert und gefriergetrocknet. Ausb. 455 mg (89 %).
$C_{20}H_{28}N_6O_8S$ (512.547)
$[\alpha]_D^{23}$ = -26,0° (c=1, in Wasser)

Beispiel 3:

Acetyl-Leu-Glu-Asn-Tyr-Cys-Asn-OH

Zu einer Lösung von 2,0 g (1,56 mmol) H-Leu-Glu(OtBu)-Asn-Tyr(tBu)-Cys(Trt)-Asn-OtBu·trifluoracetat (Liebigs Ann. Chem. 1979, 243) in 30 ml Dimethylformamid gibt man 0,4 ml N-Ethylmorpholin und 0,53 g Acetyl-N-hydroxysuccinimid. Nach 4 Stdn. Reaktionszeit bei Raumtemperatur wird im Hochvakuum eingeengt. Der Rückstand wird in Essigester gelöst und nacheinander mit ges. NaHCO$_3$-Lösung, KHSO$_4$-Lösung und Wasser ausgeschüttelt. Dabei fällt ein Niederschlag aus, der abgesaugt wird. Ausb. 1,3 g. Die Essigesterphase wird über Na$_2$SO$_4$ getrocknet und eingeengt. Der Rückstand wird mit Diethylether verrieben und abgesaugt. Ausb. 0,8 g. Gesamtausbeute 2,1 g (>100 %).
Die 1,3 g (ca. 1,07 mmol) der oben gewonnenen reinen Charge Ac-Leu-Glu(OtBu)-Asn-Tyr(tBu)-Cys(Trt)-Asn-OtBu werden in einer Mischung von 30 ml Trifluoressigsäure und 30 ml Ethylmercaptan gelöst. Nach 4 Stdn. Reaktionszeit wird die Mischung in 300 ml Wasser geschüttet und die wässrige Lösung 3 mal mit Diethylether extrahiert. Die wässrige Phase wird gefriergetrocknet. Ausb. 740 mg (87 %).
$C_{33}H_{48}N_8O_{13}S$ (796,86)
$[\alpha]_D^{23}$ = -25,1° (c=1, in Wasser).

Beispiel 4:

Synthese von H-Tyr(Bu$^t$)-Gln-Leu-Glu(OBu$^t$)-Asn-Tyr(Bu$^t$)-Cys(Trt)-Asn-OBu$^t$·HBr (7·HBr)

4a. Ddz-Tyr(Bu$^t$)-Gln-ONb (16)

Zu einer Lösung von 25,24 g (55 mmol) Ddz-Tyr(Bu$^t$)-OH, 15,88 g (50 mmol) H-Gln-ONb·HCl und 6,75 g 1-Hydroxybenzotriazol-hydrat in 100 ml N,N-Dimethylformamid gibt man bei -3°C 6,4 ml (50 mmol) N-Ethylmorpholin und 10,5 g Dicyclohexylcarbodiimid. Man läßt 1 h bei 0°C und 6 h bei Raumtemperatur rühren und über Nacht bei Raumtemperatur stehen. Der Niederschlag wird abgesaugt und das Filtrat eingeengt. Das resultierende Öl wird in Essigester gelöst, die Lösung nacheinander mit NaHCO$_3$-Lösung, Citratpuffer (pH 3) und Wasser, gewaschen, über Na$_2$SO$_4$ getrocknet und eingeengt. Das ölige Produkt wird mit Petrolether pulvrig gerieben und abgesaugt. Dann wird 3 mal mit je 100 ml Diisopropylether gekocht und dekantiert. Zum Schluß wird mit kaltem Diisopropylether verrieben, abgesaugt und mit Petrolether gewaschen. Ausb. 32,8 g (91 %); Schmp. 80 - 90°C $[\alpha]_D^{22}$ = + 15,1° (c = 1, in Methanol).

| C$_{37}$H$_{46}$N$_4$O$_{11}$ (722,81) | | | |
|---|---|---|---|
| Ber. | C 61,48 | H 6,41 | N 7,75 |
| Gef. | C 61,3 | H 6,7 | N 7,9 |

4b. Ddz-Tyr(Bu$^t$)-Gln-OH·Dicyclohexylamin

Zu einer Lösung von 32,5 g (45 mmol) 16 in 500 ml Methanol gibt man 5 ml Wasser sowie Pd/BaSO$_4$ und hydriert 7 h. Danach wird von Katalysator abgesaugt und das Filtrat eingeengt. Das zurückbleibende Öl wird in 250 ml Essigester gelöst. Dazu gibt man 11,3 ml (55 mmol) Dicyclohexylamin, läßt einige Stunden bei 3°C stehen und saugt den Niederschlag ab. Er wird mit Essigester in der Reibschale verrieben, abgesaugt und i. Vak. getrocknet. Ausb. 27 g (78 %); Schmp. 170 - 171°C, $[\alpha]_D^{23}$ = +10,2° (c = 1, in Methanol).

| C$_{42}$H$_{64}$N$_4$O$_9$ (769,0) | | | |
|---|---|---|---|
| Ber. | C 65,6 | H 8,39 | N 7,28 |
| Gef. | C 65,4 | H 8,5 | N 7,3 |

4c. Ddz-Tyr(Bu$^t$)-Gln-OH (17)

2,9 g (3,7 mmol) Ddz-Tyr(Bu$^t$)-Gln-OH·Dicyclohexylamin werden zwischen Essigester und Citratpuffer (pH 3) verteilt. Die Essigesterphase wird mit Wasser neutral gewaschen, über Na$_2$SO$_4$ getrocknet und eingeengt. Es bleibt amorphes 17 zurück.
Ausb. 2 g (90 %); Schmp. 110 - 115°C, $[\alpha]_D^{22}$ = + 19,8 (c = 1, in Methanol).

| C$_{30}$H$_{41}$N$_3$O$_9$ (587,68) | | | |
|---|---|---|---|
| Ber. | C 61,31 | H 7,03 | N 7,15 |
| Gef. | C 60,6 | H 7,2 | N 7,0 |

4d. Ddz-Tyr(Bu$^t$)-Gln-Leu-Glu(OBu$^t$)-Asn-Tyr(Bu$^t$)-Cys(Trt)-Asn-OBu$^t$ (18)

9,7 g (16,5 mmol) 17, 19,2 g (15 mmol) der Verbindung aus Beispiel 3 und 2,025 g (15 mmol) HOBt werden in 30 ml N,N-Dimethylformamid unter Rühren bei Raumtemperatur gelöst. Man kühlt auf 0°C, versetzt mit 1,95 ml (15 mmol) N-Ethylmorpholin sowie einer Lösung von 3,3 g (16 mmol) Dicyclohexylcarbodiimid in 9 ml N,N-Dimethylformamid, läßt 1 h bei 0°C sowie 4 h bei Raumtemperatur rühren, über Nacht bei Raumtemperatur stehen und saugt den Dicyclohexylharnstoff ab. Dann wird 2mal mit je 4,5 ml N,N-Dimethylformamid gewaschen. Das Filtrat läßt man unter Rühren zu 159 ml gesättigter NaHCO$_3$-Lösung fließen und rührt so lange, bis ein pulvriger Niederschlag entstanden ist. Dieser wird abgesaugt, mit Citratpuffer (pH 3) verrieben, abgesaugt, mit Wasser neutral gewaschen und bei ca. 0,1 Torr getrocknet (Ausb. 23,1 g). Die Rohsubstanz wird auf dem Dampfbad bis fast zum Sieden erwärmt, die dünne Suspension über Nacht bei 3°C aufbewahrt, der Niederschlag abgesaugt und mit Essigester sowie Ether gewaschen. Ausb. 20 g (76,8 %), $[\alpha]_D^{22}$ = -10,2° (c = 1, in Methanol).
Die Substanz zersetzt sich ab 205°C und verkohlt bei ca. 250°C. - Aminosäureanalyse: Asp 2,00; Glu 2,01;

Cys 0,75; Leu 0,99; Tyr 1,95

| $C_{92}H_{123}N_{11}O_{20}S$ (1735,15) | | | | |
|---|---|---|---|---|
| Ber. | C 63,68 | H 7,15 | N 8,88 | S 1,85 |
| Gef. | C 62,0 | H 7,2 | N 8,6 | S 2,1 |

4e. H-Tyr(Bu$^t$)-Gln-Leu-Glu(OBu$^t$)-Asn-Tyr(Bu$^t$)-Cys(Trt)-Asn-OBu$^t$)$_{\cdot HBr\ (7 \cdot HBr)}$

3,5 g (2 mmol) 18 werden in einer Mischung aus 1,75 ml Trifluoressigsäure (20 mmol), 0,35 ml Wasser und 33 ml Methylendichlorid (ca. 35 ml einer 5 % Trifluoressigsäure-Lösung mit 1 % Wasser) und 3,5 ml Anisol unter Rühren gelöst. Man läßt 3 h bei Raumtemperatur rühren, versetzt mit 2 ml (24,8 mmol) Pyridin und engt bei ca. 0,1 Torr ein. Der Rückstand wird mit Ether verrieben, abgesaugt, mit Ether gewaschen, getrocknet, mit Wasser verrieben, abgesaugt, mit Wasser gewaschen und über $P_2O_5$ getrocknet (Ausb. 3,35 g). Zur weiteren Reinigung wird die Substanz 2mal mit je 20 ml Essigester kurz zum Sieden erhitzt und heiß abgesaugt. Dann wird mit Ether nachgewaschen. Ausb. 3,0 g (92 %); Schmp. 255 - 265°C (Zers.), $[\alpha]_D^{22}$ = -20,2° (c = 1, in Methanol). Aminosäureanalyse: Asp 1,97; Glu 2,00; Cys 0,61; Leu 1,00; Tyr 2,01.

| $C_{80}H_{110}BrN_{11}O_{16}S$ (1593,8) | | | | |
|---|---|---|---|---|
| Ber. | C 60,23 | H 6,96 | N 9,67 | S 2,01 |
| Gef. | C 60,6 | H 7,0 | N 9,5 | S 2,2 |

Beispiel 5:

Die biologische Aktivität der erfindungsgemäßen Peptide der Formel I und II wird an Hand von präparativen Fettzellen und Diaphragma-Stücken aus der Ratte bestimmt. Der Begriff Tripeptid steht für Tyr Cys Asn-OH und Hexapeptid steht für Acetyl-Leu Glu Asn Tyr Cys Asn-OH. Der Begriff "basal" steht für Aktivität ohne Stimulation, Insulin steht für Humaninsulin und dpm steht für radioaktive Zerfälle pro Minute. Der Begriff Peptid steht allgemein für ein erfindungsgemäßes Peptid mit insulinartiger Wirkung.

Die Präparation von Fettzellen aus der Ratte erfolgte wie folgt:

Fettgewebe des Nebenhodens (Wistar-Ratte, 160-180 g, keine Futterbeschränkung) wird mit Kollagenase verdaut und die entstandenen vereinzelten Fettzellen durch Flotation mehrmals gewaschen.

Präparation von Diaphragma-Stücken aus der Ratte: Aus mehrmals gewaschenen Hemi-Diaphragmen (Wistar-Ratte, 60-70 g, keine Futterbeschränkung) werden kleine Gewebsstücke (5 mm Durchmesser) gestanzt.

Die beiden folgenden Teste erfassen die insulin-stimulierbare Glukose-Aufnahme, welche die funktionelle Insulin-Signalübertragungskaskade und den Glukose-Transport erfordert, unabhängig davon, ob die Glukose oxidativ (Glykolyse, Pentosephosphatweg) oder nicht-oxidativ metabolisiert wird. Die Umwandlung in Lipide, Glykogen oder membranundurchlässige Zwischenprodukte (Glukose-6-Phosphat), aber nicht die Entstehung von Laktat wird verfolgt.

a) Ratten-Fettzellen werden in Gegenwart oder Abwesenheit von Insulin oder Peptid mit D-[U-$^{14}$C]Glukose (22 μM Endkonz. D-Glukose) inkubiert. Die Zellen werden über Zentrifugation durch eine Silikonölschicht vom Medium getrennt, reisoliert und die zellassoziierte Radioaktivität bestimmt.

b) Diaphragma-Stücke werden in Gegenwart oder Abwesenheit von Insulin oder Peptid mit D-[U-$^{14}$C]Glukose (75 μM Endkonz. D-Glukose) inkubiert. Das Medium wird abgesaugt. Die Gewebsstücke werden mehrmals gewaschen und nachfolgend zur Radioaktivitätsbestimmung durch Alkali-Behandlung solubilisiert.

Die Tabelle 1 zeigt die Ergebnisse.

Tabelle 1:

| Glukoseaufnahme | | | | | |
|---|---|---|---|---|---|
| b) Diaphargma | | | a) Fettzellen | | |
| Insulin [dpm] | Tripeptid [dpm] | Hexapeptid [dpm] | Insulin [dpm] | Tripeptid [dpm] | Hexapeptid [dpm] |
| 0,1 mM | 7591 | 7429 | | 1838 | 1488 |

Tabelle 1:  (fortgesetzt)

| Glukoseaufnahme | | | | | | |
|---|---|---|---|---|---|---|
| | b) Diaphargma | | | a) Fettzellen | | |
| | Insulin [dpm] | Tripeptid [dpm] | Hexapeptid [dpm] | Insulin [dpm] | Tripeptid [dpm] | Hexapeptid [dpm] |
| 0,5 mM | | 8540 | 7752 | | 2947 | 1917 |
| 1 mM | | 11588 | 8597 | | 6533 | 4218 |
| 0,5 ng | 10055 | | | 4379 | | |
| 5 ng | 20841 | | | 26312 | | |
| basal | 7348 | | | 1235 | | |

Beispiel 6: Glucosetransport

Fettzellen und Diaphragma-Stücke werden wie in Beispiel 5 hergestellt. Die nachfolgenden Teste erfassen ausschließlich den insulin-stimulierbaren spezifischen Glukose-Transport (erleichterte Diffusion) über die Plasmamembran mittels Glukose-Carrier einschließlich der Insulin-Signalübertragungskaskade. Ein Einfluß des Glukose-Stoffwechsels auf den Glukose-Transport wird durch Verwendung des nicht-metabolisierbaren Glucose-Analogons ausgeschlossen.

a) Ratten-Fettzellen werden in Gegenwart oder Abwesenheit von Insulin oder Peptid mit 2-Deoxy-D-[1-$^3$H]Glukose (0,2 mM Endkonz. D-Glukose) und L-[1-$^{14}$C]Glukose (nicht transportierbar) inkubiert. Zur Radioaktivitätsbestimmung ([$^3$H] und [$^{14}$C]) werden die Zellen über Zentrifugation durch eine Ölschicht vom Medium getrennt. Der spezifische stereoselektive Glukose-Transport wird als Differenz zwischen der gesamten zellgebundenen Radioaktivität ([$^3$H]Glukose) und der durch Diffusion und unspezifischen Einfluß assoziierten Radioaktivität ([$^{14}$C]Glukose) berechnet.

b) Diaphragmastücke werden in Gegenwart oder Abwesenheit von Insulin oder Peptid mit 2-Desoxy-D-[1-$^3$H]Glukose (0,1 mM Endkonz. D-Glukose) und L-[1-$^{14}$C]Glukose inkubiert. Die Gewebestücke werden durch schnelle Filtration über Glasfaserfilter vom Medium abgetrennt und intensiv gewaschen. Die Radioaktivität wird in einem alkalischen Extrakt gemessen. Tabelle 2 zeigt die Ergebnisse.

Tabelle 2:

| Glukosetransport | | | | | | |
|---|---|---|---|---|---|---|
| | a) Fettzellen | | | b) Diaphragma | | |
| | Insulin [dpm] | Tripeptid [dpm] | Hexapeptid [dpm] | Insulin [dpm] | Tripeptid [dpm] | Hexapeptid [dpm] |
| 0,1 mM | | 2289 | 2218 | | 8836 | 8450 |
| 0,5 mM | | 3055 | 2842 | | 9822 | 8928 |
| 1 mM | | 5781 | 3922 | | 11252 | 9676 |
| 0,5 ng | 4851 | | | 10828 | | |
| 5 ng | 20342 | | | 17750 | | |
| basal | 2311 | | | 8522 | | |

Beispiel 7: Veresterung in vitro

Dieser Test mißt die insulin-stimulierbare Veresterung des Glyzerin-3-Phosphats in Lipid-Produkte (Triglyzeride, Phospholipde). Daran beteiligt sind die Enzyme der Lipidsynthese (z.B. Acyl-CoA: L-Glycerin-3-Phosphat Acyltransferase) einschließlich einer funktionellen Insulin-Signalübertragungskaskade. Glukose-Transport spielt dabei keine Rolle, wie die fehlende Hemmung der Veresterung durch Cytochalasin B beweist.

Ratten-Fettzellen, hergestellt wie in Beispiel 5, werden mit D-Glukose (33 µM Endkonz.) in Gegenwart oder Abwesenheit von Insulin oder Peptid inkubiert und nachfolgend zur Permeabilisierung der Plasmamembran (ohne Zerstörung von internen Membranen) mit niedrigen Konzentrationen an Saponin behandelt. Nach Zugabe von L-

[U-$^{14}$C]Glycerin-3-Phosphat wird die Inkubation fortgesetzt. Toluol-löslicher Szintillationscocktail wird hinzugefügt und das Lipid von der wäßrigen Phase durch Zentrifugation abgetrennt. Die das Lipid enthaltende Toluolphase wird entfernt und ihre Radioaktivität bestimmt. Tabelle 3 zeigt die Ergebnisse.

Tabelle 3

| Veresterung mit Fettzellen | | |
|---|---|---|
| | Insulin [dpm] | Tripeptid [dpm] |
| 0,1 mM | | 3710 |
| 0,5 mM | | 4422 |
| 1 mM | | 5398 |
| 0,5 ng | - | |
| 5 ng | 3640 | |
| basal | 3842 | |

Beispiel 8: Lipogenese

Ratten-Fettzellen aus Beispiel 5 werden zur proteolytischen Inaktivierung des Insulinrezeptors mit niedrigen Konzentrationen an Trypsin behandelt. Nach Zugabe von Proteaseinhibitoren werden die Zellen zweimal durch Flotation gewaschen und die Inkubation für 15 min bei 37°C fortgesetzt. Diese Zellen werden sodann für den Test auf Stimulation der Lipogenese durch die Peptide benutzt. Eine Kontrollinkubation mit Insulin zeigt, daß die trypsin-behandelten Zellen nur eine sehr geringe insulin-stimulierbare Lipogenese und damit nur eine recht begrenzte Zahl an funktionellen Insulinrezeptoren (im Hinblick auf Insulinbindung) aufweisen. Damit mißt dieser Test eine Stimulation der Lipogenese durch Eingriffe in die Insulin-Signalübertragungskaskade nach der Rezeptorbindung von Insulin. Tabelle 4 zeigt die Ergebnisse.

Tabelle 4

| Lipogenese | | |
|---|---|---|
| | Insulin [dpm] | Tripeptid [dpm] |
| 0,1 mM | | 3250 |
| 0,5 mM | | 4145 |
| 1 mM | | 5072 |
| 0,5 ng | - | |
| 5 ng | 3087 | |
| basal | 2355 | |

Beispiel 9: Blutglucoseprofil in Mäusen

Weibliche Charles River Wiga Balb-C-Mäuse mit einem Gewicht von 17 bis 21 g (Alter etwa 30 Tage) erhalten eine Standard-Diät. 16 Stunden lang vor Versuchsbeginn erhalten die Mäuse keine Nahrung. Je 5 Tieren pro Versuchsgruppe wird intravenös eine wäßrige Lösung (pH 6) der Verbindung aus Beispiel 4 (Oktapeptid) appliziert. Applikationsvolumen 0,3 ml/Tier.
Die Tabelle 5 zeigt Blutglucosewerte in Prozent als Differenz zwischen der Kontrollgruppe (5 Tiere, Pufferlösung pH 6,0; Applikationsvolumen 0,3 ml/Tier) und den Tieren mit dem erfindungsgemäßen Oktapeptid. Es wird jeweils der Durchschnittswert für jede Versuchsgruppe angegeben.

Tabelle 5

| Blutglucoseprofil | | |
|---|---|---|
| Zeit [Minuten] | Oktapeptid [%] | |
| | 500 µg/Tier | 1000 µg/Tier |
| 20 | - 2 | -31 |
| 40 | - 5 | -19 |
| 60 | -13 | -23 |

Tabelle 5   (fortgesetzt)

| Blutglucoseprofil | | |
|---|---|---|
| Zeit [Minuten] | Oktapeptid [%] | |
| | 500 µg/Tier | 1000 µg/Tier |
| 75 | -21 | -24 |
| 90 | -21 | -19 |

**Patentansprüche**

**Patentansprüche für folgende Vertragsstaaten : AT, BE, CH, DE, DK, FR, GB, IT, LI, LU, NL, SE**

1. Peptid der Formel II,

$$X\text{-}Q\text{-}Cys\text{-}D \qquad\qquad (II)$$

wobei

Q    für Tyr, Tyr($R^1$), Phe($R^2$), Trp($R^2$) oder Nal,
D    für Asp, Asn, D-Asp, D-Asn, beta-Ala oder Azagly-$NH_2$ und
X    für

    a) Wasserstoff,
    b) $R^1$-CO,
    c) ($C_1$-$C_{18}$)-Alkoxy-CO, d) ($C_3$-$C_{18}$)-Cycloalkoxy-CO oder e) ($C_6$-$C_{14}$)Aryl-($C_1$-$C_8$)-Alkoxy-CO, steht,

$R^1$    für 1.1 ($C_1$-$C_{18}$)-Alkyl,

    1.2 ($C_3$-$C_{18}$)-Cylcoalkyl,
    1.3 ($C_6$-$C_{14}$)-Aryl,
    1.4 ($C_6$-$C_{14}$)-Aryl, ein- oder mehrfach substituiert durch 1.4.1 ($C_1$-$C_8$)-Alkyl oder
    1.5 ($C_1$-$C_8$)-Alkyl, ein oder mehrfach substituiert durch 1.5.1 ($C_6$-$C_{14}$)-Aryl, steht,

$R^2$    für

    2.1 ($C_1$-$C_{18}$)-Alkyl,
    2.2 ($C_3$-$C_{18}$)-Cycloalkyl,
    2.3 ($C_1$-$C_{18}$)-Alkoxy,
    2.4 ($C_3$-$C_{14}$)-Cycloalkoxy,
    2.5 ($C_6$-$C_{14}$)-Aryl,
    2.6 ($C_6$-$C_{14}$)-Aryl, ein- oder mehrfach substituiert durch 2.6.1 ($C_1$-$C_8$)-Alkyl,
        2.6.2 ($C_1$-$C_8$)-Alkoxy,
    2.7 ($C_6$-$C_{14}$)-Aryloxy,
    2.8 ($C_6$-$C_{14}$)-Aryloxy, ein- oder mehrfach substituiert durch 2.8.1 ($C_1$-$C_8$)-Alkyl,
        2.8.2 ($C_1$-$C_8$)-Alkoxy,
    2.9 ($C_1$-$C_8$)-Alkyl, ein- oder mehrfach substituiert durch 2.9.1 ($C_6$-$C_{14}$)-Aryl,
    2.10 ($C_6$-$C_8$)-Alkoxy, ein- oder mehrfach substituiert durch 2.10.1 ($C_6$-$C_{14}$)-Aryl,
    2.11 Halogen wie Fluor, Chlor, Brom oder Jod,
    2.12 Nitro oder
    2.13 Trifluormethyl, steht, oder

Dimere der Peptide der Formel I mit Cystin als Dimerisierungs-Komponente, ihre gegebenenfalls stereoisomeren Formen oder physiologisch verträgliche Salze des Peptids der Formel I, und die Verbindung Asn-Tyr-Cys-Asn-OH.

2. Peptid der Formel II, nach Anspruch 1 mit der Sequenz Tyr-Cys-Asn.

3. Pharmazeutische Zubereitung, gekennzeichnet durch einen wirksamen Gehalt an mindestens einem Peptid der Formel II gemäß einem oder mehreren der Ansprüche 1 oder 2 in gelöster, amorpher oder kristalliner Form.

4. Pharmazeutische Zubereitung gemäß Anspruch 3, gekennzeichnet durch einen Gehalt an mindestens einem modifizierten oder unmodifizierten Insulin(derivat).

5. Verfahren zur Herstellung einer pharmazeutischen Zubereitung gemäß Anspruch 3 oder 4, dadurch gekennzeichnet, daß man mindestens eine Verbindung der Formel II, mit einem physiologisch annehmbaren Träger sowie gegebenenfalls geeigneten Zusatz- und/oder Hilfsstoffen in eine geeignete Darreichungsform bringt.

6. Verfahren gemäß Anspruch 5, dadurch gekennzeichnet, daß man mindestens ein modifiziertes oder unmodifiziertes Insulin(derivat) einsetzt.

7. Verfahren zur Herstellung eines Peptids der Formel II gemäß der Ansprüche 1 oder 2, dadurch gekennzeichnet, daß man

a) ein Segment mit C-terminaler freier Carboxylgruppe oder dessen aktiviertes Derivat mit einem entsprechenden Segment mit N-terminaler freier Aminogruppe umsetzt oder
b) das Peptid stufenweise aufbaut, und in der nach (a) oder (b) erhaltenen Verbindung gegebenenfalls eine oder mehrere zum Schutz anderer Funktionen temporär eingeführte Schutzgruppen abspaltet.

8. Verwendung einer Verbindung der Formel II gemäß der Ansprüche 1 oder 2 zur Herstellung einer pharmazeutischen Zubereitung zur Behandlung des Diabetes mellitus oder nicht insulinabhängiger Diabetes.


**Patentansprüche für folgende Vertragsstaaten : ES, GR**

1. Verfahren zur Herstellung eines Peptids der Formel II,

$$X\text{-}Q\text{-}Cys\text{-}D \qquad\qquad (II)$$

wobei

Q   für Tyr, Tyr($R^1$), Phe($R^2$), Trp($R^2$) oder Nal,
D   für Asp, Asn, D-Asp, D-Asn, beta-Ala oder Azagly-$NH_2$ und
X   für

a) Wasserstoff,
b) $R^1$-CO,
c) $(C_1\text{-}C_{18})$-Alkoxy-CO,
d) $(C_3\text{-}C_{18})$-Cycloalkoxy-CO oder
e) $(C_6\text{-}C_{14})$Aryl-$(C_1\text{-}C_8)$-Alkoxy-CO, steht

$R^1$   für

1.1 $(C_1\text{-}C_{18})$-Alkyl,
1.2 $(C_3\text{-}C_{18})$-Cycloalkyl,
1.3 $(C_6\text{-}C_{14})$-Aryl,
1.4 $(C_6\text{-}C_{14})$-Aryl, ein- oder mehrfach substituiert durch 1.4.1 $(C_1\text{-}C_8)$-Alkyl, oder
1.5 $(C_1\text{-}C_8)$-Alkyl, ein oder mehrfach substituiert durch 1.5.1 $(C_6\text{-}C_{14})$-Aryl, steht,

$R^2$   für

2.1 $(C_1\text{-}C_{18})$-Alkyl,
2.2 $(C_3\text{-}C_{18})$-Cycloalkyl,

2.3 (C$_1$-C$_{18}$)-Alkoxy,
2.4 (C$_3$-C$_{14}$)-Cycloalkoxy,
2.5 (C$_6$-C$_{14}$)-Aryl,
2.6 (C$_6$-C$_{14}$)-Aryl, ein- oder mehrfach substituiert durch 2.6.1 (C$_1$-C$_8$)-Alkyl,
     2.6.2 (C$_1$-C$_8$)-Alkoxy,
2.7 (C$_6$-C$_{14}$)-Aryloxy,
2.8 (C$_6$-C$_{14}$)-Aryloxy, ein- oder mehrfach substituiert durch 2.8.1 (C$_1$-C$_8$)-Alkyl,
     2.8.2 (C$_1$-C$_8$)-Alkoxy,
2.9 (C$_1$-C$_8$)-Alkyl, ein- oder mehrfach substituiert durch 2.9.1 (C$_6$-C$_{14}$)-Aryl,
2.10 (C$_6$-C$_8$)-Alkoxy, ein- oder mehrfach substituiert durch 2.10.1 (C$_6$-C$_{14}$)-Aryl,
2.11 Halogen wie Fluor, Chlor, Brom oder Jod,
2.12 Nitro oder
2.13 Trifluormethyl, steht, oder

Dimere der Peptide der Formel I mit Cystin als Dimerisierungs-Komponente, ihre gegebenenfalls stereoisomeren Formen oder physiologisch verträgliche Salze des Peptids der Formel I, und der Verbindung Asn-Tyr-Cys-Asn-OH, dadurch gekennzeichnet, daß man

    a) ein Segment mit C-terminaler freier Carboxylgruppe oder dessen aktiviertes Derivat mit einem entsprechenden Segment mit N-terminaler freier Aminogruppe umsetzt oder
    b) das Peptid stufenweise aufbaut, und in der nach (a) oder (b) erhaltenen Verbindung gegebenenfalls eine oder mehrere zum Schutz anderer Funktionen temporär eingeführte Schutzgruppen abspaltet.

**2.** Verfahren nach Anspruch 1 bis 3, dadurch gekennzeichnet, daß man ein Peptid der Formel II mit der Sequenz Tyr-Cys-Asn herstellt.

**3.** Verfahren zur Herstellung einer pharmazeutischen Zubereitung, dadurch gekennzeichnet, daß man eine wirksame Menge an mindestens einer Verbindung der Formel II, mit einem physiologisch annehmbaren Träger sowie gegebenenfalls geeigneten Zusatz- und/oder Hilfsstoffen in eine geeignete Darreichungsform bringt.

**4.** Verfahren nach Anspruch 3, dadurch gekennzeichnet, daß man der pharmazeutischen Zubereitung noch mindestens ein modifiziertes oder unmodifiziertes Insulin(derivat) zusetzt.

**5.** Verwendung einer Verbindung der Formel II gemäß der Ansprüche 1 oder 2 zur Herstellung einer pharmazeutischen Zubereitung zur Behandlung des Diabetes mellitus oder nicht insulinabhängiger Diabetes.

**Claims**

**Claims for the following Contracting States : AT, BE, CH, DE, DK, FR, GB, IT, LI, LU, NL, SE**

**1.** A peptide of the formula II

$$X\text{-}Q\text{-}Cys\text{-}D \qquad (II)$$

where

Q is     Tyr, Tyr(R$^1$), Phe(R$^2$), Trp(R$^2$) or Nal,
D is     Asp, Asn, D-Asp, D-Asn, beta-Ala or Azagly- NH$_2$, and
X is     a) hydrogen,
       b) R$^1$-CO,
       c) (C$_1$-C$_{18}$)-alkoxy-CO,
       d) (C$_3$-C$_{18}$)-cycloalkoxy-CO or e) (C$_6$-C$_{14}$)-aryl-(C$_1$-C$_8$)-alkoxy-CO,

R$^1$ is     1.1 (C$_1$-C$_{18}$)-alkyl,
       1.2 (C$_3$-C$_{18}$)-cycloalkyl,

1.3 $(C_6$-$C_{14})$-aryl,
1.4 $(C_6$-$C_{14})$-aryl substituted one or more times by
    1.4.1 $(C_1$-$C_8)$-alkyl or
1.5 $(C_1$-$C_8)$-alkyl substituted one or more times by
    1.5.1 $(C_6$-$C_{14})$-aryl,

$R^2$ is    2.1 $(C_1$-$C_{18})$-alkyl,

2.2 $(C_3$-$C_{18})$-cycloalkyl,
2.3 $(C_1$-$C_{18})$-alkoxy,
2.4 $(C_3$-$C_{14})$-cycloalkoxy,
2.5 $(C_6$-$C_{14})$-aryl,
2.6 $(C_6$-$C_{14})$-aryl substituted one or more times by
    2.6.1 $(C_1$-$C_8)$-alkyl,
    2.6.2 $(C_1$-$C_8)$-alkoxy,
2.7 $(C_6$-$C_{14})$-aryloxy,
2.8 $(C_6$-$C_{14})$-aryloxy substituted one or more times by
    2.8.1 $(C_1$-$C_8)$-alkyl,
    2.8.2 $(C_1$-$C_8)$-alkoxy,
2.9 $(C_1$-$C_8)$-alkyl substituted one or more times by
    2.9.1 $(C_6$-$C_{14})$-aryl,
2.10 $(C_6$-$C_8)$-alkoxy substituted one or more times by
    2.10.1 $(C_6$-$C_{14})$-aryl,
2.11 halogen such as fluorine, chlorine, bromine or iodine,
2.12 nitro or
2.13 trifluoromethyl, or

dimers of the peptides of the formula II with cystine as dimerization component, the stereoisomeric forms thereof, where appropriate, or physiologically tolerated salts of the peptide of the formula II, and the compound Asn-Tyr-Cys-Asn-OH.

**2.** A peptide of the formula II as claimed in claim 1 with the sequence Tyr-Cys-Asn.

**3.** A pharmaceutical composition which has an effective content of at least one peptide of the formula II as claimed in one or more of claims 1 and 2 in dissolved, amorphous or crystalline form.

**4.** A pharmaceutical composition as claimed in claim 3, which contains at least one modified or unmodified insulin or derivative thereof.

**5.** A process for the preparation of a pharmaceutical composition as claimed in claim 3 or 4, which comprises converting at least one compound of the formula II, with a physiologically acceptable vehicle and, where appropriate, suitable additives and/or auxiliaries, into a suitable dosage form.

**6.** The process as claimed in claim 5, wherein at least one modified or unmodified insulin or derivative thereof is employed.

**7.** A process for the preparation of a peptide of the formula II as claimed in claim 1 or 2, which comprises

a) reacting a segment with C-terminal free carboxyl group or the activated derivative thereof with a corresponding segment with N-terminal free amino group or
b) synthesizing the peptide stepwise, and eliminating, in the compound obtained as in (a) or (b), where appropriate, one or more protective groups temporarily introduced to protect other functions.

**8.** The use of a compound of the formula II as claimed claim 1 or 2 for the preparation of a pharmaceutical composition for the treatment of diabetes mellitus or non-insulin-dependent diabetes.

**EP 0 491 361 B1**

**Claims for the following Contracting States : ES, GR**

1. A process for the preparation of a peptide of the formula II

$$X\text{-}Q\text{-}Cys\text{-}D \qquad\qquad\qquad (II)$$

where

Q is Tyr, Tyr ($R^1$), Phe($R^2$), Trp($R^2$) or Nal,

D is Asp, Asn, D-Asp, D-Asn, beta-Ala or Azagly- $NH_2$, and

X is
    a) hydrogen,
    b) $R^1$-CO,
    c) ($C_1$-$C_{18}$) -alkoxy-CO,
    d) ($C_3$-$C_{18}$)-cycloalkoxy-CO or
    e) ($C_6$-$C_{14}$)-aryl-($C_1$-$C_8$)-alkoxy-CO,

$R^1$ is
    1.1 ($C_1$-$C_{18}$)-alkyl,
    1.2 ($C_3$-$C_{18}$)-cycloalkyl,
    1.3 ($C_6$-$C_{14}$)-aryl,
    1.4 ($C_6$-$C_{14}$)-aryl substituted one or more times by
        1.4.1 ($C_1$-$C_8$)-alkyl or
    1.5 ($C_1$-$C_8$)-alkyl substituted one or more times by
        1.5.1($C_6$-$C_{14}$)-aryl,

$R^2$ is
    2.1 ($C_1$-$C_{18}$)-alkyl,
    2.2 ($C_3$-$C_{18}$)-cycloalkyl,
    2.3 ($C_1$-$C_{18}$)-alkoxy,
    2.4 ($C_3$-$C_{14}$)-cycloalkoxy,
    2.5 ($C_6$-$C_{14}$)-aryl,
    2.6 ($C_6$-$C_{14}$)-aryl substituted one or more times by
        2.6.1 ($C_1$-$C_8$)-alkyl,
        2.6.2 ($C_1$-$C_8$)-alkoxy,
    2.7 ($C_6$-$C_{14}$)-aryloxy,
    2.8 ($C_6$-$C_{14}$)-aryloxy substituted one or more times by
        2.8.1 ($C_1$-$C_8$)-alkyl,
        2.8.2 ($C_1$-$C_8$)-alkoxy,
    2.9 ($C_1$-$C_8$)-alkyl substituted one or more times by
        2.9.1 ($C_6$-$C_{14}$)-aryl,
    2.10 ($C_6$-$C_8$)-alkoxy substituted one or more times by
        2.10.1 ($C_6$-$C_{14}$)-aryl,
    2.11 halogen such as fluorine, chlorine, bromine or iodine,
    2.12 nitro or
    2.13 trifluoromethyl, or

dimers of the peptides of the formula II with cystine as dimerization component, the stereoisomeric forms thereof, where appropriate, or physiologically tolerated salts of the peptide of the formula II, and of the compound Asn-Tyr-Cys-Asn-OH, which comprises

    a) reacting a segment with C-terminal free carboxyl group or the activated derivative thereof with a corresponding segment with N-terminal free amino group or
    b) synthesizing the peptide stepwise, and eliminating, in the compound obtained as in (a) or (b), where appropriate, one or more protective groups temporarily introduced to protect other functions.

2. The process as claimed in claim 1, wherein a peptide of the formula II with the sequence Tyr-Cys-Asn is prepared.

3. A process for the preparation of a pharmaceutical composition, which comprises converting an effective amount of at least one compound of the formula II, with a physiologically acceptable vehicle and, where appropriate, suitable additives and/or auxiliaries, into a suitable dosage form.

4. The process as claimed in claim 3, wherein at least one modified or unmodified insulin or derivative thereof is also added to the pharmaceutical composition.

5. The use of a compound of the formula II as claimed in claim 1 or 2 for the preparation of a pharmaceutical composition for the treatment of diabetes mellitus or non-insulin-dependent diabetes.

**Revendications**

**Revendications pour les Etats contractants suivants : AT, BE, CH, DE, DK, FR, GB, IT, LI, LU, NL, SE**

1. Peptide de formule II

$$X\text{-}Q\text{-}Cys\text{-}D \qquad\qquad (II)$$

dans laquelle

Q    représente Tyr, Tyr($R^1$), Phe($R^2$), Trp($R^2$) ou Nal,
D    représente Asp, Asn, D-Asp, D-Asn, bêta-Ala ou Azagly-$NH_2$ et
X    représente

        a) un atome d'hydrogène
        b) $R^1$-CO
        c) (alkoxy en $C_1$-$C_{18}$)-CO,
        d) cyclo(alkoxy en $C_3$-$C_{18}$)-CO ou
        e) (aryl en $C_6$-$C_{14}$)-(alkoxy en $C_1$-$C_8$)-CO,

$R^1$    représente

        1.1 alkyle en $C_1$-$C_{18}$
        1.2 cycloalkyle en $C_3$-$C_{18}$
        1.3 aryle en $C_6$-$C_{14}$
        1.4 aryle en $C_6$-$C_{14}$, une ou plusieurs fois substitué par
            1.4.1 alkyle en $C_1$-$C_8$, ou
        1.5 alkyle en $C_1$-$C_8$, une ou plusieurs fois substitué par
            1.5.1 aryle en $C_6$-$C_{14}$,

$R^2$    représente

        2.1 alkyle en $C_1$-$C_{18}$,
        2.2 cycloalkyle en $C_3$-$C_{18}$,
        2.3 alkoxy en $C_1$-$C_{18}$,
        2.4 cycloalkoxy en $C_3$-$C_{14}$,
        2.5 aryle en $C_6$-$C_{14}$,
        2.6 aryle en $C_6$-$C_{14}$, une ou plusieurs fois substitué par
            2.6.1 alkyle en $C_1$-$C_{18}$,
            2.6.2 alkoxy en $C_1$-$C_8$,
        2.7 aryloxy en $C_6$-$C_{14}$,
        2.8 aryloxy en $C_6$-$C_{14}$, une ou plusieurs fois substitué par
            2.8.1 alkyle en $C_1$-$C_8$,
            2.8.2 alkoxy en $C_1$-$C_8$,
        2.9 alkyle en $C_1$-$C_8$, une ou plusieurs fois substitué par
            2.9.1 aryle en $C_6$-$C_{14}$,
        2.10 alkoxy en $C_6$-$C_8$, une ou plusieurs fois substitué par

2.10.1 aryle en $C_6$-$C_{14}$,

2.11 des atomes d'halogène, comme le fluor, le chlore, le brome ou l'iode,

2.12 un groupe nitro ou

2.13 un groupe trifluorométhyle, ou

des peptides dimères de formule II avec la cystine comme composant de dimérisation, leurs formes éventuellement stéréisomères ou des sels physiologiquement tolérés du peptide de formule II, et le composé Asn-Tyr-Cys-Asn-OH.

2. Peptide de formule II selon la revendication 1, avec la séquence Tyr-Cys-Asn.

3. Préparation pharmaceutique, caractérisée par une teneur efficace en au moins un peptide de formule II selon une ou plusieurs des revendications 1 et 2 sous forme dissoute, amorphe ou cristalline.

4. Préparation pharmaceutique selon la revendication 3, caractérisée par une teneur en moins une insuline (dérivé d'insuline) modifiée ou non modifiée.

5. Procédé pour préparer une préparation pharmaceutique selon la revendication 3 ou 4, caractérisé en ce que l'on met en forme d'administration appropriée au moins un composé de formule II, avec un véhicule physiologiquement toléré, ainsi qu'éventuellement avec des additifs et/ou adjuvants appropriés.

6. Procédé selon la revendication 5, caractérisé en ce que l'on utilise au moins une insuline (dérivé d'insuline) modifiée ou non modifiée.

7. Procédé pour la préparation d'un peptide de formule II selon les revendications 1 ou 2, caractérisé en ce que

a) on fait réagir un segment avec un groupe carboxy C-terminal ou son dérivé activé avec un segment correspondant avec un groupe amino N-terminal libre ou
b) on construit le peptide par étape, et on dissocie les groupes protecteurs introduits temporairement pour protéger d'autres fonctions dans le composé obtenu selon (a) ou (b).

8. Utilisation d'un composé de formule II selon la revendication 1 ou 2 pour préparer une préparation pharmaceutique pour le traitement du Diabetes mellitus ou du diabète non insuline-dépendant.

**Revendications pour les Etats contractants suivants : ES, GR**

1. Procédé de préparation d'un peptide formule II

$$X-Q-Cys-D \qquad\qquad (II)$$

dans laquelle

Q représente Tyr, Tyr($R^1$), Phe($R^2$), Trp($R^2$) ou Nal,
D représente Asp, Asn, D-Asp, D-Asn, bêta-Ala ou Azagly-$NH_2$ et
X représente

a) un atome d'hydrogène
b) $R^1$-CO
c) (alkoxy en $C_1$-$C_{18}$)-CO,
d) cyclo(alkoxy en $C_3$-$C_{13}$)-CO ou
e) (aryl en $C_6$-$C_{14}$)-(alkoxy en $C_1$-$C_8$)-CO,

$R^1$ représente

1.1 alkyle en $C_1$-$C_{18}$
1.2 cycloalkyle en $C_3$-$C_{18}$

1.3 aryle en $C_6$-$C_{14}$

1.4 aryle en $C_6$-$C_{14}$, une ou plusieurs fois substitué par

    1.4.1 alkyle en $C_1$-$C_8$, ou

1.5 alkyle en $C_1$-$C_8$, une ou plusieurs fois substitué par

    1.5.1 aryle en $C_6$-$C_{14}$,

$R^2$     représente

2.1 alkyle en $C_1$-$C_{18}$,

2.2 cycloalkyle en $C_3$-$C_{18}$,

2.3 alkoxy en $C_1$-$C_{18}$,

2.4 cycloalkoxy en $C_3$-$C_{14}$,

2.5 aryle en $C_6$-$C_{14}$,

2.6 aryle en $C_6$-$C_{14}$, une ou plusieurs fois substitué par

    2.6.1 alkyle en $C_1$-$C_{18}$,

    2.6.2 alkoxy en $C_1$-$C_8$,

2.7 aryloxy en $C_6$-$C_{14}$,

2.8 aryloxy en $C_6$-$C_{14}$, une ou plusieurs fois substitué par

    2.8.1 alkyle en $C_1$-$C_8$,

    2.8.2 alkoxy en $C_1$-$C_8$,

2.9 alkyle en $C_1$-$C_8$, une ou plusieurs fois substitué par

    2.9.1 aryle en $C_6$-$C_{14}$,

2.10 alkoxy en $C_6$-$C_8$, une ou plusieurs fois substitué par

    2.10.1 aryle en $C_6$-$C_{14}$,

2.11 des atomes d'halogène, comme le fluor, le chlore, le brome ou l'iode,

2.12 un groupe nitro ou

2.13 un groupe trifluorométhyle, ou

des peptides dimères de formule II avec la cystine comme composant de dimérisation, leurs formes éventuellement stéréisomères ou des sels physiologiquement tolérés du peptide de formule II, et le composé Asn-Tyr-Cys-Asn-OH, caractérisé en ce que

a) on fait réagir un segment avec un groupe carboxy C-terminal ou son dérivé activé avec un segment correspondant avec un groupe amino N-terminal libre ou

b) on construit le peptide par étape, et on dissocie les groupes protecteurs introduits temporairement dans le composé obtenu selon (a) ou (b) pour protéger d'autres fonctions.

2. Procédé selon la revendication 1, caractérisé en ce que l'on prépare un peptide de formule II avec la séquence Tyr-Cys-Asn.

3. Procédé pour préparer une préparation pharmaceutique, caractérisé en ce que l'on met en forme d'administration appropriée une quantité efficace d'au moins un composé de formule II, avec un véhicule physiologiquement toléré, ainsi qu'éventuellement avec des additifs et/ou adjuvants appropriés.

4. Procédé selon la revendication 3, caractérisé en ce que l'on ajoute à la préparation pharmaceutique encore au moins une insuline (dérivé d'insuline) modifiée ou non modifiée.

5. Utilisation d'un composé de formule II selon les revendications 1 ou 2 pour préparer une préparation pharmaceutique pour le traitement du Diabetes mellitus ou du diabète non insulino-dépendant.